# EUROPÄISCHE PATENTSCHRIFT

(11) **EP 1 786 313 B1**
(45) Veröffentlichungstag und Bekanntmachung des Hinweises auf die Patenterteilung: **30.11.2011**
(21) Anmeldenummer: 05785748.4
(22) Anmeldetag: 30.08.2005
(51) Int. Cl.: A61B 3/103

(54) **SCHNELLE WELLENFRONTMESSUNG**
RAPID WAVEFRONT MEASUREMENT
RAPIDE MESURE DE FRONTS D'ONDES

(30) Priorität: 01.09.2004 EP 04020783
(43) Veröffentlichungstag der Anmeldung: 23.05.2007
(73) Patentinhaber: WaveLight GmbH, 91058 Erlangen (DE)
(72) Erfinder: GORSCHBOTH, Claudia, 90411 Nürnberg (DE); DONITZKY, Christof, 90542 Eckental (DE)
(74) Vertreter: von Hellfeld, Axel
(86) Internationale Anmeldenummer: PCT/EP2005/009354
(87) Internationale Veröffentlichungsnummer: WO 2006/024504

(56) Entgegenhaltungen:
- DE-A- 10 154 194
- US-A1- 2003 071 969
- US-B1- 6 199 986
- US-B1- 6 338 559
- HOFER H ET AL: "DYNAMICS OF THE EYE'S WAVE ABERRATION" JOURNAL OF THE OPTICAL SOCIETY OF AMERICA - A, OPTICAL SOCIETY OF AMERICA, WASHINGTON, US, Bd. 18, Nr. 3, März 2001 (2001-03), Seiten 497-506, XP001041247 ISSN: 1084-7529

## Beschreibung

### Gebiet der Erfindung

Die vorliegende Erfindung betrifft im Allgemeinen die Erfassung von Wellenfronten zur Bestimmung der Aberration eines Auges und insbesondere die schnelle, dynamische Erfassung von Wellenfronten, um nichtlineare Abbildungseigenschaften einer Linse für ein Auge zu bestimmen.

### Hintergrund der Erfindung

In der Ophthalmologie sind Systeme zur Messung der Wellenfrontaberration eines Auges bekannt. In der Praxis haben sich zur Wellenfrontmessung sogenannte Hartmann-Shack-Sensoren etabliert. Bei diesen Sensoren wird die vermessende Wellenfront von einem Mikrolinsenarray auf einen lichtempfindlichen Detektor als Punktmuster abgebildet. Bei Abweichungen der detektierten Wellenfront von einer idealen Wellenfront aufgrund der Aberration des Auges kann diese aus dem detektierten Punktmuster berechnet werden. Hierfür wird ein schmales Lichtbündel auf das zu überprüfende Auge gerichtet und Licht nach Wechselwirkung mit dem Auge auf den Hartmann-Shack-Sensor abgebildet.

Insbesondere im Bereich der refraktiven Chirurgie kommen Systeme zur Messung von Wellenfrontaberrationen zum Einsatz. Dabei ist es bekannt, Systeme zu verwenden, die die Aberration eines Auges messen; wenn das Auge einen in einer festgelegten Entfernung erscheinenden Stimulus fixiert. Neuere Systeme erlauben die Berechnung der Aberration eines Auges durch Erfassung von Aberrationen bei Fixierung eines Stimulus, der in unterschiedlichen Entfernungen wahrgenommen wird. Die Berechnung der Aberration erfolgt auf der Grundlage der für unterschiedliche Stimuli erfassten Aberrationen.

Dokument DE-A-101 54 194 offenbart den nächstliegenden Stand der Technik.

### Aufgabe der Erfindung

Aufgabe der vorliegenden Erfindung ist es, Lösungen bereitzustellen, die eine verbesserte und umfassenderere Messung von Wellenfronten im Allgemeinen zur Bestimmung der Aberration eines Auges und insbesondere zur Bestimmung optischer Eigenschaften von Linsen für ein Auge ermöglichen.

### Zusammenfassung der Erfindung

Zur Lösung der Aufgabe stellt die vorliegende Erfindung ein Verfahren, eine Vorrichtung sowie Verwendungen gemäß den unabhängigen Ansprüchen bereit. Vorteilhafte Weiterbildungen erfindungsgemäßer Lösungen sind in den abhängigen Ansprüchen definiert.

Gemäß Anspruch 1 stellt die vorliegende Erfindung eine Vorrichtung zur Messung von Wellenfronten einer Linse für ein Auge bereit. Dabei kann unter einer Linse für ein Auge insbesondere die Linse eines Auges, eine Kontaktlinse oder eine intraokulare Linse verstanden werden.

Die Vorrichtung umfasst eine Strahlungsquelle zur Abgabe von Messstrahlung, die auf die Linse zu richten ist. Ferner ist eine Sensoreinrichtung vorhanden, um Wellenfronten in zu der Sensoreinrichtung gelangender Messstrahlung zu erfassen, die sich durch die Messstrahlung der Strahlungsquelle nach Wechselwirkung mit der Linse ergibt.

Die Sensoreinrichtung ist so ausgeführt, dass sie die einfallende Messstrahlung mit einer Abtastfrequenz nach Wellenfronten abtastet, die wenigstens so groß wie die Frequenz ist, mit der Wellenfrontänderungen in der einfallenden Messstrahlung auftreten.

Dadurch wird beispielsweise erreicht, dass auch in dynamischen Sehsituationen die Aberration eines Auges korrekt bestimmt werden kann. Des Weiteren können auf diese Weise Akkommodationsvorgänge eines Auges in bisher nicht bekanntem Umfang analysiert werden. Des Weiteren wird dadurch ermöglicht, wie im Folgenden detaillierter ausgeführt, die chromatische Aberration einer Linse für ein Auge zu bestimmen.

Die Strahlungsquelle ist so ausgeführt, dass sie eine Messstrahlung abgibt, deren Wellenlänge sich mit einer Strahlungsabgabefrequenz ändert. Darunter ist insbesondere zu verstehen, dass sich die Wellenlänge der von der Strahlungsquelle abgegebenen Messstrahlung nach einer vorgegebenen Zeitdauer ändert. Die Abtastfrequenz der Sensoreinrichtung ist wenigstens so groß wie die Strahlungsabgabefrequenz.

Die Vorrichtung kann ferner eine Stimuluserzeugungseinrichtung umfassen, um einen Stimulus zu erzeugen, der dynamische Änderungen der Linse bewirken soll. Beispiele für dynamische Änderungen umfassen Änderungen der Linse aufgrund von Akkommodationen. Vorzugsweise ist hierbei die Abtastfrequenz wenigstens so groß wie die Frequenz der zu bewirkenden Änderungen. Z. B. kann hierbei eine Abtastfrequenz gewählt werden, die wenigstens so groß wie die Frequenz ist, mit der der Stimulus variiert wird.

Vorzugsweise umfasst die Sensoreinrichtung einen optischen Sensor, der beispielsweise ein CMOS-Sensor sein kann.

Die Sensoreinrichtung kann eine Abtastfrequenz von wenigstens 70 Hertz, 100 Hertz oder größer aufweisen.

Die Sensoreinrichtung kann eine Verstärkungseinrichtung aufweisen, um auf die Sensoreinrichtung einfallende Messstrahlung, d.h. Messstrahlung der Strahlungsquelle nach Wechselwirkung mit der Linse, zu verstärken. Beispielsweise kann die Verstärkungseinrichtung einen Bildverstärker umfassen.

Vorzugsweise ist die Verstärkungseinrichtung so angeordnet, dass die Verstärkung der einfallenden Messstrahlung vor deren Erfassung mit der Abtastfrequenz erfolgt.

Die Sensoreinrichtung kann eine Linsenanordnung umfassen, die beispielsweise in Abhängigkeit einer gewünschten Auflösung und/oder einer gewünschten Dynamik der Sensoreinrichtung insgesamt ausgelegt ist.

Vorzugsweise ist die Strahlungsquelle so ausgeführt, dass deren Messstrahlung eine vorgegebene maximale Strahlungsleistung aufweist, die für die Linse vorgegeben ist. Insbesondere wenn es sich bei der Linse um die Linse eines Auges handelt, werden durch diese Ausführungsform unerwünschte Einflüsse aufgrund der Messstrahlung vermieden.

Die Strahlungsquelle kann wenigstens eine Quelle für Laserstrahlung umfassen, die vorzugsweise eine fest vorgegebene Wellenlänge abgibt. Beispielsweise kann die wenigstens eine Laserstrahlenquelle ein Laser, eine Laserdiode oder eine Superlimiszenzdiode (SLD) sein.

Die Strahlungsquelle kann ausgangsseitig mit einer Schalteinrichtung verbunden sein, die mit einer Schaltfrequenz betrieben werden kann. Diese Ausführungsform ist insbesondere dann bevorzugt, wenn mehr als eine Laserstrahlenquelle verwendet wird, um Laserstrahlung der unterschiedlichen Laserstrahlenquellen gemäß der Schaltfrequenz der Schalteinrichtung auf die Linse zu richten. Bei Verwendung von nur einer Laserstrahlenquelle kann deren Laserstrahlung gemäß der Schaltfrequenz, beispielsweise zu vorbestimmten Zeitpunkten, in vorbestimmten, regelmäßigen oder unregelmäßigen Zeitabständen, auf die Linse gerichtet werden.

Vorzugsweise umfasst die Schalteinrichtung einen Faserkoppler.
Gemäß einer Ausführungsform ist die Strahlungsquelle eingerichtet, Messstrahlung mit einer, zwei oder mehreren Wellenlängen im Bereich zwischen 400 nm und 1000 nm zu erzeugen. Dadurch ist es beispielsweise möglich, schnelle Wellenfrontmessungen bei verschiedenen, diskreten Wellenlängen durchzuführen, die über den gesamten sichtbaren Bereich bis in den Infrarotbereich hinein reichen.

Als Stimuluserzeugungseinrichtung ist vorzugsweise ein Badaloptometer vorgesehen.

Des Weiteren stellt die vorliegende Erfindung ein Verfahren zur Messung von Wellenfronten einer Linse für ein Auge bereit, das die Schritte umfasst, einen Messstrahl auf die Linse zu richten und Wellenfronten von Messstrahlung nach Wechselwirkung mit der Linse zu erfassen, wobei die Erfassung der Messstrahlung nach Wechselwirkung mit der Linse mit einer Abtastfrequenz erfolgt, die wenigstens so groß wie die Frequenz ist, mit der Wellenfrontänderungen in der erfassten Messstrahlung auftreten.

Des Weiteren stellt die vorliegende Erfindung Verwendungen der oben beschriebenen Vorrichtung in einer ihrer Ausführungsformen bereit, um dynamische Akkommodationsänderungen der Linse eines Auges, chromatischen Aberrationen eines Auges oder die Dispersion einer Kontaktlinse oder einer intraokularen Linse für ein Auge oder optische Variationen des Tränenfilms zu messen.

### Kurzbeschreibung der Zeichnungen

Bei der folgenden Beschreibung wird auf die beigefügten Zeichnungen Bezug genommen, die zeigen:
Fig. 1 eine schematische Darstellung einer bevorzugten Ausführungsform der erfindungsgemäßen Vorrichtung zur Messung von Wellenfronten unter dynamischen Sehbedingungen,
Fig. 2 eine schematische Darstellung einer bevorzugten Ausführungsform der erfindungsgemäßen Vorrichtung zur Erfassung von Wellenfronten für verschiedene Wellenlängen unter einer statischen Sehbedingung, und
Fig. 3 eine schematische Darstellung einer bevorzugten Ausführungsform zur Erfassung von Wellenfronten einer Kontaktlinse oder einer intraokularen Linse für ein Auge.

### Beschreibung bevorzugter Ausführungsformen

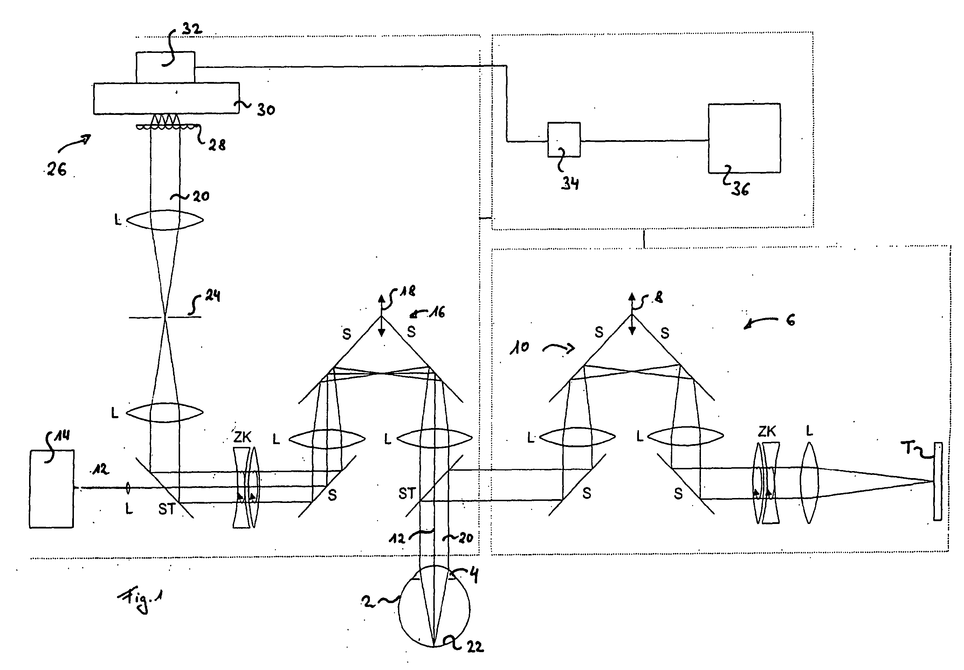

In Fig. 1 bis 3 sind Linsen L, Spiegel S, Strahlteiler ST und Zylinderkompensatoren ZK mit den genannten Bezugszeichen ohne weitere Differenzierungen angegeben.

Die in Fig. 1 schematisch dargestellte Ausführungsform dient zur Erfassung von Wellenfronten eines Auges 2 und insbesondere einer Linse 4 des Auges 2. Insbesondere dient diese Ausführungsform zur Messung von Wellenfronten unter dynamischen Sehbedingungen für das Auge 2 in Form von dynamischen Akkommodationsvorgängen.

Um dynamische Akkommodationen des Auges 2 bzw. der Linse 4 zu bewirken, wird ein in unterschiedlichen Entfernungen erscheinender Stimulus bereitgestellt. Hierfür wird über eine im ganzen mit 6 bezeichnete Stimuluserzeugungseinrichtung in Form eines Badaloptometers ein als Stimulus wahrnehmbares Bild eines Ziels oder Targets T bereitgestellt. Das Bild des Targets T wird dem Auge über Linsen L, einen Zylinderkompensator ZK, Spiegel S und einen Strahlteiler ST bereitgestellt. Um den Stimulus bzw. das Bild des Targets T in für das Auge unterschiedlichen Entfernungen erscheinen zu lassen, umfasst die Stimuluserzeugungseinrichtung 6 eine in Richtung des Pfeils 8 bewegbare Spiegel- oder Prismenanordnung 10.

Zur Messung wird Messstrahlung 12, hier in Form von Laserstrahlung eines Lasers 14 verwendet. Der Laser 14 kann beispielsweise ein Laser, eine Laserdiode oder eine Superluminiszenzdiode (SLD) sein und Messstrahlung 12 abgeben, die eine Wellenlänge im Bereich sichtbaren Lichts bis in den Infrarotbereich hinein aufweist.

Die Messstrahlung wird über Linsen L, eine Zylinderkompensator ZK und Spiegel S dem Auge zugeführt. Diese Anordnung optischer Komponenten umfasst eine Spiegel- oder Prismenanordnung 16, die in Richtung des Pfeils 18 bewegbar ist. Bewegungen der Anordnungen 10 und 16 erfolgen im Allgemeinen in Abhängigkeit von einander, um einerseits das Bild des Targets T in unterschiedlichen Entfernungen erscheinen zu lassen und andererseits die sich daraus ergebenen Auswirkungen für die Messstrahlung und/oder das Auge hinsichtlich der Meßstrahlung zu berücksichtigen.

Messstrahlung 20, die sich nach Wechselwirkung der Messstrahlung 12 mit dem Auge 2 (insbesondere Wechselwirkungen aufgrund von Durchtritten durch die Linse 4 und Reflexion an der Retina 22 des Auges 2) ergibt, wird über Linsen L, Spiegel S, einen Zylinderkompensator ZK und eine Blende 24 zu einer im ganzen mit 26 bezeichneten Sensoreinrichtung geführt. Die Sensoreinrichtung 26 dient zur Erfassung von Wellenfronten der Messstrahlung 20.

Die Sensoreinrichtung 26 umfasst eine Linsenanordnung 28, beispielsweise in Form eines Mikrolinsenarrays. Die Linsenanordnung 28 kann zum Beispiel Linsen mit einem Durchmesser von 650 µm und einer Brennweite von 30 mm aufweisen.

Der Linsenanordnung 28 nachgeordnet ist eine Verstärkungseinrichtung 30. Die Verstärkungseinrichtung 30, beispielsweise in Form eines Bildverstärkers, verstärkt die Messstrahlung 20 nach Abbildung durch die Linsenanordnung 28 auf entsprechende Bereiche der Verstärkungseinrichtung 30.

Die Verwendung der Verstärkungseinrichtung 30 ermöglicht es, als Messstrahlung 12 eine Strahlung zu verwenden, deren Strahlungsleistung für das Auge 2 maximale Grenzwerte nicht überschreitet. Dies führt im Allgemeinen zu Messstrahlung 12 relativ geringer Strahlungsleistung. Nach Wechselwirkung der Messstrahlung 12 mit dem Auge 2 ergibt sich eine Messstrahlung 20 noch geringerer Strahlungsleistung. Üblicherweise wird dieses Problem dadurch gelöst, dass einerseits möglichst lange Belichtungszeiten und andererseits möglichst lichtempfindliche Sensoren verwendet werden. Lange Belichtungszeiten verbieten es, dynamische Sehvorgänge zu analysieren. Lichtempfindliche Sensoren stellen nur geringe Aufnahmefrequenzen bereit. Die Verstärkungseinrichtung 30 ermöglicht es demgegenüber einerseits die für das Auge 2 zulässigen Strahlungsleistungsgrenzwerte nicht zu überschreiten und andererseits schnellere Sensoren zur Erfassung von Wellenfronten zu verwenden, die eine geringe Lichtempfindlichkeit aufweisen. Insbesondere dient die Verstärkereinrichtung 30 dazu, einem Sensor 32 verstärkte, aus der Messstrahlung 20 resultierende Strahlung bereitzustellen, so dass ein für Signalauswertungen ausreichender Signal-Rausch-Abstand erreicht wird.

Der Sensor 32 ist vorzugsweise ein CMOS-Sensor mit Bildraten von bis zu 500 Bildern pro Sekunde und mehr.

Insbesondere ist vorgesehen, dass der Sensor 32 Messungen mit einer Frequenz von mehr als 100 Hertz ermöglicht. Hierfür sind aufgrund ihrer hohen Bildraten CMOS-Sensoren besonders geeignet.

Der Sensor 32 gibt erfasster Messstrahlung entsprechende Signale aus, die an eine Auswerteeinrichtung 34 weitergeleitet werden. Als Auswerteeinrichtung 34 kann beispielsweise ein digitaler Signalprozessor verwendet werden.

Insbesondere ist es vorgesehen, dass die Linsennordung 28 und der Sensor 32 einen Hartmann-Shack-Sensor darstellen. Dabei wird von der Linsenanordnung 28 ein Punktmuster auf den Sensor 32 abgebildet, das Informationen über Wellenfronten der Messstrahlung enthält.

Zur Steuerung der Vorrichtung von Fig. 1 ist eine Steuereinrichtung 36 vorgesehen. Die Steuereinrichtung 36 kann einen Personal Computer, einen Mikroprozessor und dergleichen umfassen. Die Steuereinrichtung 36 steuert insbesondere den Betrieb der gesamten Vorrichtung von Fig. 1 einschließlich der Stimuluserzeugungseinrichtung 6 und der Sensoreinrichtung 26.

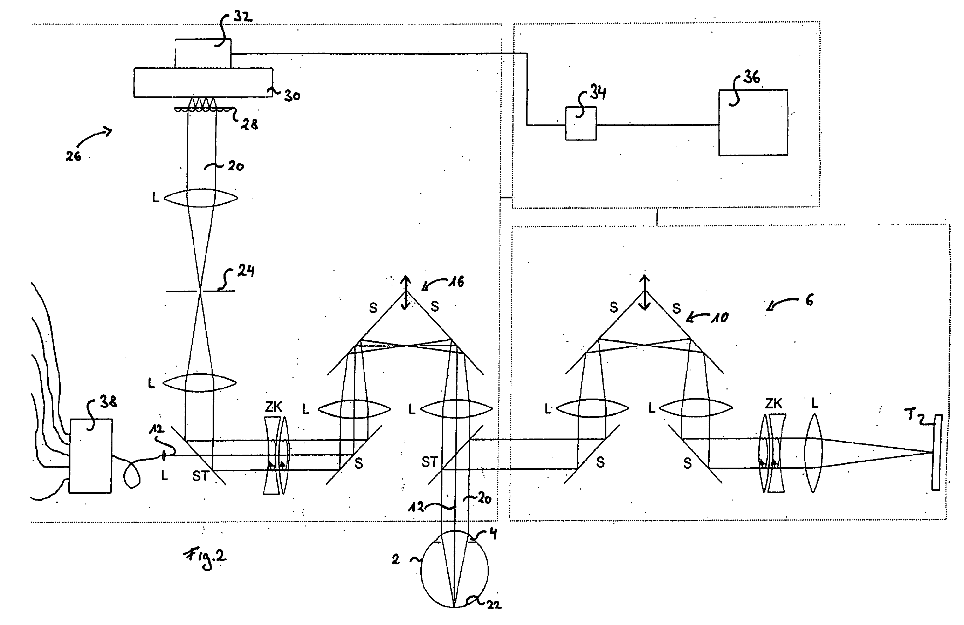

Die in Fig. 2 schematische Darstellung zeigt eine Ausführungsform, die sich von der Ausführungsform gemäß Fig. 1 wie im Folgenden ausgeführt unterscheidet. Bei beiden Ausführungsformen verwendete Komponenten sind mit gleichen Bezugszeichen angegeben.

Die auch bei der Ausführungsform von Fig. 2 vorgesehene Bewegbarkeit der Spiegel- oder Prismenanordnungen 10 und 16 kann bei den im Folgenden beschriebenen Messungen, insbesondere auch bei statischen Messungen, zur Vorkompensation von Fehlsichtigkeit verwendet werden.

Die Ausführungsform von Fig. 2 verwendet zur Erzeugung der Messstrahlung 12 mehrere Strahlungsquellen 14₁ - 14ₙ unterschiedlicher, diskreter Wellenlängen. In Fig. 2 sind beispielhaft fünf solcher Strahlungsquellen 14 dargestellt. Beispielsweise können die Strahlungsquellen 14 Strahlung in einem Wellenlängenbereich über den gesamten sichtbaren Bereich bis in den nahen Infrarotbereich hinein abgeben. Die Strahlungsquellen 14 können z. B. Laser, Laserdioden und/oder SLDs sein.

Von den Strahlungsquellen 14 abgegebene Strahlung wird zu einer Schalteinrichtung 38 übertragen. Die Übertragung von Strahlung der Strahlungsquellen 14 zu der Schalteinrichtung 38 kann beispielsweise über optische Faserleiter erfolgen.

Die Schalteinrichtung 38, beispielsweise in Form eines sogenannten Faserschalters , wird mit einer Schaltfrequenz betrieben, um Strahlung der Strahlungsquellen 14 zu unterschiedlichen Zeitpunkten und/oder in unterschiedlichen zeitlichen Abständen und/oder für unterschiedliche Zeitdauern als Messstrahlung 12 auszugeben. Die Abfolge, in der Strahlung der Strahlungsquellen 14 als Messstrahlung 12 abgegeben wird, kann beispielsweise bei der kleinsten (größten) Wellenlänge beginnen und bis zur größten (kleinsten) Wellenlänge fortschreiten, um dann wieder mit der kleinsten (größten) Wellenlänge zu beginnen. Es ist auch möglich, dass die Abfolge, in der Strahlung der Strahlungsquellen 14 als Messstrahlung 12 abgegeben wird, in beliebiger, chaotischer Abfolge durchgeführt wird.

Eine mögliche Anwendung der Vorrichtung von Fig. 2 ist die Erfassung der chromatischen Aberration des Auges 2 bzw. der Linse 4. Hierfür kann dem Auge 2 von der Stimuluserzeugungseinrichtung 6 ein als ortsfest wahrnehmbarer Stimulus bereitgestellt werden. Dabei werden dem Auge 2 Strahlungen der Strahlungsquellen 14 über die Schalteinrichtung 38 als Messstrahlung 12 zugeführt. Die Schalteinrichtung 38 wird mit einer Schaltfrequenz betrieben, die hoch genug ist, um von einem statischen Zustand des Auges 2 und insbesondere der Linse 4 ausgehen zu können. Auch wenn die Stimuluserzeugungseinrichtung 6 einen ortsfest erscheinenden Stimulus bereitstellt, ist das Auge bei Fixierung eines solchen Stimulus gewissen in geringem Maße dynamischen Änderungen unterworfen, wie zum Beispiel Mikrosakkaden. Um den Einfluss solcher Änderungen auszuschließen, wird die Schalteinrichtung 38 mit einer entsprechend hohen Schaltfrequenz betrieben. Beispielsweise kann die Schaltfrequenz 100 Hertz betragen.

Messstrahlung 20 nach Wechselwirkung mit dem Auge 2 bzw. der Linse 4 wird, wie oben unter Bezugnahme auf Fig. 1 beschrieben, der Sensoreinrichtung 26 zugeführt. Hier umfasst die Messstrahlung 20 Strahlungen unterschiedlicher Wellenlängen, nämlich Wellenlängen der Strahlungsquellen 14. Um Wechselwirkungen der Messstrahlung 12 jeweils für die unterschiedlichen Wellenlängen der Strahlungsquellen 14 zu erfassen, wird die Sensoreinrichtung 26 und insbesondere der Sensor 32 bei der Vermessung von Wellenfronten mit einer Abtastfrequenz (entsprechend der Messung einer Wellenfront) betrieben, die wenigstens so groß wie die Schaltfrequenz der Schaltreinrichtung 38 ist.

Aufgrund der Wahl der Schaltfrequenz der Schalteinrichtung 38 geben von der Sensoreinrichtung 26 erfasste Wellenfronten der Messstrahlung 20 für die unterschiedlichen Wellenlängen die chromatische Aberration des Auges 2 bzw. der Linse 4 an.

Wird die Sensoreinrichtung 26 mit einer ausreichend hohen Abtastfrequenz betrieben, ist es möglich, die Vorrichtung von Fig. 2 sowohl zur Erfassung akkommodationsabhäniger Charakteristika des Auges 2 als auch zur Messung von dessen chromatischer Aberration zu verwenden. Für eine solche Anwendung ist vorgesehen, die Stimuluserzeugungseinrichtung 6 so zu betreiben, dass durch in unterschiedlichen Abständen erscheinende Stimuli dynamische Akkommodationsvorgänge bewirkt werden. Um dabei auch von Wellenlängen abhängige Charakteristika des Auges 2 zu ermitteln, wird die Schalteinrichtung 38 mit einer derart hohen Schaltfrequenz betrieben, dass für wenigstens einen Akkommodationszustand, vorteilhafterweise für mehrere oder jeden Akkommodationszustand, hinsichtlich der unterschiedlichen Wellenlängen von einem statischen Zustand des Auges ausgegangen werden kann.

Um die dann in der Messstrahlung 20 vorliegende Information zu detektieren, sind die Sensoreinrichtung 26 und insbesondere der Sensor 32 mit einer Abtastfrequenz zu betreiben, die einem ganzzahligen Vielfachen des Produktes der Frequenzen entspricht, mit denen die Stimuluserzeugungseinrichtung 6 und die Schalteinrichtung 38 betrieben werden.

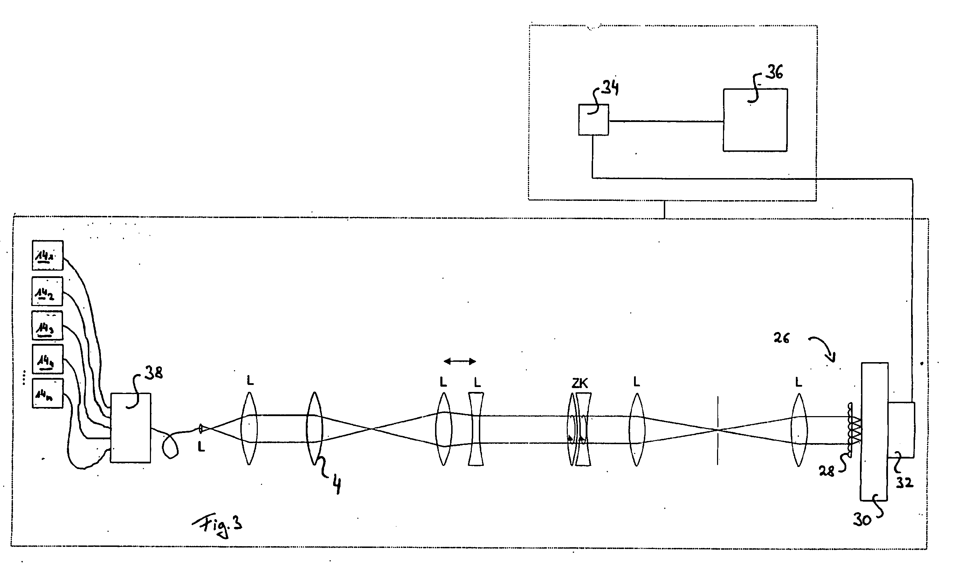

Die in Fig. 3 schematisch dargestellte Ausführungsform wird zur Messung der Dispersion einer Linse für ein Auge verwendet. Die Linse 4 kann beispielsweise eine Kontaktlinse oder intraokulare Linse sein. Vergleichbar zu der Ausführungsform von Fig. 2 umfasst die Ausführungsform von Fig. 3 Strahlungsquellen 14₁ - 14ₙ, die Strahlung unterschiedlicher Wellenlängen abgeben. Die in diesem Zusammenhang unter Bezugnahme auf Fig. 2 gemachten Ausführungen gelten hier entsprechend. Dies gilt auch für die Strahlung von den Strahlungsquellen 14 erhaltende Schalteinrichtung 38. Von der Schalteinrichtung 38 abgegebene Messstrahlung 12 wird direkt oder über zwischen der Schalteinrichtung 38 und der Linse 4 angeordnete Optiken, die in Fig. 3 lediglich zur Veranschaulichung eine Austrittslinse L und eine Linse Lₛ zur Strahlaufweitung umfassen, der Linse 4 zugeführt. Messstrahlung 20, die sich aus Wechselwirkung der Messstrahlung 12 mit der Linse 4 ergibt, wird über beispielhaft Linsen L, und einem Zylinderkompensator ZK (insbesondere zur Vorkomensation) sowie eine Blende BI mit nachgeschalteter Linse L umfassende Optiken einer Sensoreinrichtung 26 zugeführt.

Die Sensoreinrichtung 26 ist mit den Sensoreinrichtungen 26 von Fig. 1 und 2 im Wesentlichen vergleichbar. Insbesondere gilt dies für die Abtastraten, mit denen die Sensoreinrichtung 26 betrieben wird.

Mit der Ausführungsform von Fig. 3 ist es möglich, die Dispersion, d.h. die wellenlängenabhängige Brechung von Licht durch die Linse 4 zu ermitteln. Auch im Fall von Kontaktlinsen und intraokularen Linsen wird die Sensoreinrichtung 26 mit einer wie oben ausgeführt hohen Abtastfrequenz zu betreiben, was den Vorteil hat, dass die Überprüfung wellenlängenabhängiger Eigenschaften von Linsen für Augen, beispielsweise im Rahmen der industriellen Fertigung von Kontaktlinsen, besonders schnell durchgeführt werden kann. Mit der Vorrichtung von Fig. 3 ist es ferner möglich, die Qualitätskontrolle zu verbessern.

Bei der obigen Beschreibung der Ausführungsformen von Fig. 1 und 2 wird davon ausgegangen, dass es sich bei der Linse 4 des Auges 2 um dessen eigene Linse handelt. Es ist allerdings auch möglich, die Ausführungsformen von Fig. 1 und 2 zur Messung an einem Auge zu verwenden, das zusätzlich eine Kontaktlinse aufweist oder bei dem eine intraokulare Linse eingesetzt ist. Auch ist es möglich, die Vorrichtung von Fig. 1 und 2 zur Messung an einem Auge zu verwenden, das einer refraktiven chirurgischen Behandlung unterzogen wird.

## Patentansprüche

1. Vorrichtung zur Messung von Wellenfronten, die von einer Linse für ein Auge erzeugt werden, mit:
- einer Strahlungsquelle (14) zur Abgabe von auf die Linse (4) zu richtender Messstrahlung (12), und
- einer Sensoreinrichtung (26) zur Erfassung von Wellenfronten von einfallender Messstrahlung (20) nach Wechselwirkung mit der Linse (4), wobei
- die Sensoreinrichtung (26) die einfallende Messstrahlung (20) mit einer Abtastfrequenz erfaßt, die wenigstens so groß wie die Frequenz ist, mit der Wellenfrontänderungen in der einfallenden Messstrahlung (20) auftreten und
- die Strahlungsquelle (14) Messstrahlung (12) mit einer Strahlungsabgabefrequenz und sich ändernder Wellenlänge abgibt, derart, dass die Abtastfrequenz wenigstens so groß wie die Strahlungsabgabefrequenz ist

2. Vorrichtung nach Anspruch 1, mit
- einer Stimuluserzeugungseinrichtung (6) zur Erzeugung eines dynamische Änderungen der Linse bewirkenden Stimulus, wobei die Abtastfrequenz wenigstens so groß wie die Frequenz der zu bewirkenden Änderungen ist, und/oder
- einem Badaloptometer als Stimuluserzeugungseinrichtung (6) zur Erzeugung eines dynamische Änderungen der Linse bewirkenden Stimulus, wobei die Abtastfrequenz wenigstens so groß wie die Frequenz der zu bewirkenden Änderungen ist.

3. Vorrichtung nach einem der vorherigen Ansprüche, bei der
- die Sensoreinrichtung (26) einen optischen Sensor (32) umfasst, und/oder
- die Sensoreinrichtung (26) einen CMOS-Sensor als optischen Sensor (32) umfasst, und/oder
- die Sensoreinrichtung (26) eine Abtastfrequenz von wenigstens 70 Hertz aufweist, und/oder
- die Sensoreinrichtung (26) eine Verstärkungseinrichtung (30) zur Verstärkung einfallender Messstrahlung (20) umfasst, und/oder
- die Sensoreinrichtung (26) einen Bildverstärker als Verstärkungseinrichtung (30) zur Verstärkung einfallender Messstrahlung (20) umfasst, und/oder
- die Sensoreinrichtung (26) eine Verstärkungseinrichtung (30) zur Verstärkung einfallender Messstrahlung (20) umfasst, wobei die Verstärkungseinrichtung (30) zur Verstärkung einfallender Messstrahlung (20) vor deren Erfassung mit der Abtastrate angeordnet ist, und/oder
- die Sensoreinrichtung (26) eine Linsenanordnung (28) umfasst, und/oder
- die Linsenanordnung (28) in Abhängigkeit einer gewünschten Auflösung und/oder einer gewünschten Dynamik der Sensoreinrichtung (26) ausgelegt ist, und/oder
- die Strahlungsquelle (14) zur Abgabe von Messstrahlung (12) mit einer für die Linse (4) und/oder das Auge (2) vorgegebenen maximalen Strahlungsleistung ausgelegt ist, und/oder
- die Strahlungsquelle (14) wenigstens eine Laserstrahlenquelle, z.B. ein Laser, eine Laserdiode und/oder eine Supertuminiszenzdiode ist, und/oder
- die Strahlungsquelle (14) zur Abgabe von Messstrahtung mit wenigstens einer Wellenlänge im Bereich zwischen 400 nm und 1000 nm ausgelegt ist.

4. Vorrichtung nach einem der vorherigen Ansprüche, bei der
- die Strahlungsquelle (14) ausgangsseitig mit einer Schalteinrichtung (38) verbunden ist, die eine Schaltfrequenz aufweist, wobei die Abtastfrequenz wenigstens so groß wie die Schaltfrequenz ist, und/oder
- die Strahlungsquelle (14) ausgangsseitig mit einem Faserschalter und/oder einem Faserkoppler als Schalteinrichtung (38) verbunden ist, die eine Schaltfrequenz aufweist, wobei die Abtastfrequenz wenigstens so groß wie die Schaltfrequenz ist umfasst.

5. Verfahren zur Messung von Wellenfronten, die von einer Linse für ein Auge erzeugt werden, mit folgenden Schritten:
- Aussenden von Messstrahlung auf die Linse, und
- Erfassen von Wellenfronten von Messstrahlung nach Wechselwirkung mit der Linse, wobei
- die Erfassung von Messstrahlung nach Wechselwirkungen mit der Linse mit einer Abtastfrequenz erfolgt, die wenigstens so groß wie die Frequenz ist, mit der Wellenfrontänderungen in der Messstrahlung nach Wechselwirkung mit der Linse auftreten, und
- die Messstrahlung mit einer Frequenz von 100 Hertz oder mehr getaktet ist, und
- eine Messstrahlung mit sich mit einer Strahlungsabgabefrequenz ändernden Wellenlängen erzeugt und auf die Linse ausgesendet wird, und
- die Abtastung bei der Erfassung von Messstrahlungsweltenfronten mit einer Abtastfrequenz erfolgt, die wenigsten so groß wie die Strahlungsabgabefrequenz ist.

6. Verfahren nach Anspruch 5, bei der
- ein Stimulus erzeugt wird, um dynamische Änderungen der Linse zu bewirken, und
- die Erfassung der Wellenfronten mit einer Abtastfrequenz erfolgt, die wenigstens so groß wie die Frequenz der zu bewirkenden Änderungen ist.

7. Verfahren nach einem der Ansprüche 5 bis 6, bei dem
- die Erfassung von Wellenfronten mit einer Abtastfrequenz von wenigstens 70 Hertz erfolgt, und/oder
- Messstrahlung nach Wechselwirkung mit der Unse verstärkt wird, und/oder
- Messstrahlung nach Wechselwirkung mit der Unse verstärkt wird, wobei die Verstärkung vor der Erfassung mit der Abtastrate erfolgt, und/oder
- zur Erfassung von Wellenfronten Messstrahlung nach Wechselwirkung mit der Linse mittels einer Linsenanordnung abgebildet wird, und/oder
- eine Messstrahlung erzeugt wird, die eine für die Unse und/oder das Auge vorgegebene maximale Strahlungsleistung aufweist, und/oder
- zur Erzeugung der Messstrahlung wenigstens eine Laserstrahlung oder eine Superluminiszenzdiode einer vorgegebenen Wellenlänge verwendet wird, und/oder
- eine Messstrahlung mit wenigstens einer Wellenlänge im Bereich zwischen 400 nm und 1000 nm erzeugt wird.

8. Vorfahren nach einem der Ansprüche 5 bis 7, bei dem
- zur Erzeugung der Messstrahlung wenigstens zwei Laserstrahlungen oder wenigstens zwei Superluminiszenzdioden verwendet werden, die gemäß einer Schaltfrequenz auf die Unse gerichtet werden, und
- zum Erfassen von Wellenfronten eine Abtastfrequenz verwendet wird, die wenigstens so groß wie die Schaltfrequenz ist.

9. Verwendung der Vorrichtung nach einem der Ansprüche 1 bis 4 zur Messung dynamischer Akkommodationsänderungen der Unse eines Auges.

10. Verwendung der Vorrichtung nach einem der Ansprüche 1 bis 4 zur Messung chromatischer Aberrationen eines Auges.

11. Verwendung der Vorrichtung nach einem der Ansprüche 1 bis 4 zur Messung der Dispersion einer Kontaktlinse oder einer intraokularen Linse für ein Auge.

12. Verwendung der Vorrichtung nach einem der Ansprüche 1 bis 4 zur Messung optischer Einflüsse bei Bewegungen einer Kontaktlinse oder intraokularen Linse relativ zum Übrigen eines damit versehenen Auges.

13. Verwendung der Vorrichtung nach einem der Ansprüche 1 bis 4 zur Messung optischer Einflüsse von Tränenfilmänderungen.

14. Verwendung der Vorrichtung nach einem der Ansprüche 1 bis 4 zur Erfassung und/oder Korrektur von Abberationen einer intraokularen Linse eines Auges.

## Claims

1. Device for measuring wave fronts generated by a lens for an eye, with:
- a radiation source (14) for emitting test radiation (12) to be directed at the lens (4) and
- a sensor device (26) for detecting wave fronts of incident test radiation (20) after interaction with the lens (4), wherein
- the sensor device (26) detects the incident test radiation (20) at a scanning frequency which is at least equal in size to the frequency at which changes in wave fronts occur in the incident test radiation (20) and
- the radiation source (14) emits test radiation (12) with a radiation emission frequency and changing wavelength in such a way that the scanning frequency is at least equal in size to the radiation emission frequency.

2. Device according to claim 1, with
- a stimulus-generating device (6) for generating a stimulus effecting dynamic changes to the lens, the scanning frequency being at least equal in size to the frequency of the changes to be effected and/or
- a Badal optometer as stimulus-generating device (6) for generating a stimulus effecting dynamic changes to the lens, the scanning frequency being at least equal in size to the frequency of the changes to be effected.

3. Device according to one of the previous claims, in which
- the sensor device (26) comprises an optical sensor (32) and/or
- the sensor device (26) comprises a CMOS sensor as optical sensor (32) and/or
- the sensor device (26) comprises a scanning frequency of at least 70 Hertz and/or
- the sensor device (26) comprises an amplifying device (30) for amplifying incident test radiation (20) and/or
- the sensor device (26) comprises an image amplifier as amplifying device (30) for amplifying incident test radiation (20) and/or
- the sensor device (26) comprises an amplifying device (30) for amplifying incident test radiation (20), the amplifying device (30) being arranged for amplification of incident test radiation (20) before it is detected at the scanning rate and/or
- the sensor device (26) comprises a lens arrangement (28) and/or
- the lens arrangement (28) is configured as a function of a desired resolution and/or a desired dynamic of the sensor device (26) and/or
- the radiation source (14) is configured for emitting test radiation (12) with a maximum radiant power preset for the lens (4) and/or the eye (2) and/or
- the radiation source (14) is at least a laser beam source, e.g. a laser, a laser diode and/or a superluminescent diode and/or
- the radiation source (14) is configured for emitting test radiation with at least one wavelength in a range of between 400 nm and 1000 nm.

4. Device according to one of the previous claims, in which
- the radiation source (14) is connected on the output side to a switching device (38) which has a switching frequency, the scanning frequency being at least equal in size to the switching frequency and/or
- the radiation source (14) is connected on the output side to a fibre switch and/or a fibre-optic coupler as switching device (38), which has a switching frequency, the scanning frequency being at least equal in size to the switching frequency.

5. Method for measuring wave fronts generated by a lens for an eye, with the following steps:
- transmitting test radiation to the lens and
- detecting wave fronts of test radiation after interaction with the lens, wherein
- detection of the test radiation is done after interactions with the lens at a scanning frequency which is at least equal in size to the frequency at which changes in wave fronts occur in the test radiation after interaction with the lens and
- the test radiation is clocked at a frequency of 100 Hertz or more, and
- a test radiation with wavelengths changing at a radiation emission frequency is generated and transmitted to the lens and
- the scanning is done during detection of test radiation wave fronts at a scanning frequency which is at least equal in size to the radiation emission frequency.

6. Method according to claim 5, in which
- a stimulus is generated in order to effect dynamic changes to the lens and
- detection of the wave fronts is done at a scanning frequency which is at least equal in size to the frequency of the changes to be effected.

7. Method according to one of claims 5 or 6, in which
- detection of wave fronts is done at a scanning frequency of at least 70 Hertz and/or
- test radiation is amplified after interaction with the lens and/or
- test radiation is amplified after interaction with the lens, the amplification being done before detection at the scanning rate and/or
- to detect wave fronts, test radiation is imaged by a lens arrangement after interaction with the lens and/or
- a test radiation is generated which has a maximum radiant power preset for the lens and/or the eye and/or
- to generate the test radiation, at least one laser radiation or one superluminescent diode of a preset wavelength is used and/or
- a test radiation with at least one wavelength in a range of between 400 nm and 1000 nm is generated.

8. Method according to one of claims 5 to 7, in which
- to generate the test radiation, at least two laser radiations or at least two superluminescent diodes are used, which are directed at the lens according to a switching frequency and
- to detect wave fronts a scanning frequency is used which is at least equal in size to the switching frequency.

9. Use of the device according to one of claims 1 to 4 for measuring dynamic accommodation changes to the lens of an eye.

10. Use of the device according to one of claims 1 to 4 for measuring chromatic aberrations of an eye.

11. Use of the device according to one of claims 1 to 4 for measuring the dispersion of a contact lens or an intra-ocular lens for an eye.

12. Use of the device according to one of claims 1 to 4 for measuring optical influences during movements of a contact lens or intra-ocular lens relative to the rest of an eye provided therewith.

13. Use of the device according to one of claims 1 to 4 for measuring optical influences of changes in the tear film.

14. Use of the device according to one of claims 1 to 4 for detecting and/or correction of aberrations of an intra-ocular lens of an eye.

## Revendications

1. Dispositif pour la mesure de fronts d'onde produits par une lentille pour un oeil, comportant
- une source de rayonnement (14) destinée à émettre un rayonnement de mesure (12) à diriger sur la lentille (4), et
- un dispositif de détection (26) pour détecter des fronts d'onde du rayonnement de mesure incident (20) après interaction avec la lentille (4),
- le dispositif de détection (26) détectant le rayonnement de mesure incident (20) au moyen d'une fréquence de balayage qui est pour le moins égale à la fréquence à laquelle se produisent des modifications de fronts d'onde dans le rayonnement de mesure incident (20) et
- la source de rayonnement (14) émettant un rayonnement de mesure (12) à une fréquence d'émission de rayonnement et à une longueur d'onde changeante de sorte que la fréquence de balayage est pour le moins égale à la fréquence d'émission de rayonnement.

2. Dispositif selon la revendication 1, comportant
- un dispositif générateur de stimulus (6) pour générer un stimulus provoquant des modifications dynamiques de la lentille, la fréquence de balayage étant pour le moins égale à la fréquence des modifications à provoquer, et/ou
- un optomètre de Badal en tant que dispositif générateur de stimulus (6) pour générer un stimulus provoquant des modifications dynamiques de la lentille, la fréquence de balayage étant pour le moins égale à la fréquence des modifications à provoquer.

3. Dispositif selon l'une des revendications précédentes, dans le cadre duquel
- le dispositif de détection (26) comprend un capteur optique (32), et/ou
- le dispositif de détection (26) comprend un capteur CMOS en tant que capteur optique (32), et/ou
- le dispositif de détection (26) présente une fréquence de balayage d'au moins 70 Hertz, et/ou
- le dispositif de détection (26) comprend un dispositif d'amplification (30) destiné à amplifier le rayonnement de mesure incident (20), et/ou
- le dispositif de détection (26) comprend un amplificateur d'images en tant que dispositif d'amplification (30) destiné à amplifier le rayonnement de mesure incident (20), et/ou
- le dispositif de détection (26) comprend un dispositif d'amplification (30) destiné à amplifier le rayonnement de mesure incident (20), le dispositif d'amplification (30) destiné à amplifier le rayonnement de mesure incident (20) étant disposé en amont de sa détection au moyen de la fréquence de balayage, et/ou
- le dispositif de détection (26) comprend un agencement de lentilles (28), et/ou
- l'agencement de lentilles (28) est conçu en fonction d'une résolution souhaitée et/ou d'une dynamique souhaitée du dispositif de détection (26), et/ou
- la source de rayonnement (14) destinée à émettre un rayonnement de mesure (12) est conçue avec une puissance de rayonnement maximale prédéfinie pour la lentille (4) et/ou pour l'oeil (2), et/ou
- la source de rayonnement (14) est pour le moins une source de rayon laser, p. ex. un laser, une diode laser et/ou une diode superluminescente, et/ou
- la source de rayonnement (14) destinée à émettre un rayonnement de mesure est conçue pour émettre à au moins une longueur d'onde comprise entre 400 et 1000 nm.

4. Dispositif selon l'une des revendications précédentes, dans le cadre duquel
- la source de rayonnement (14) est reliée du côté de la sortie à un dispositif de commutation (38) présentant une fréquence de commutation, la fréquence de balayage étant pour le moins égale à la fréquence de commutation, et/ou
- la source de rayonnement (14) est reliée du côté de la sortie à un commutateur à fibres et/ou à un coupleur de fibres en tant que dispositif de commutation (38) présentant une fréquence de commutation, la fréquence de balayage étant pour le moins égale à la fréquence de commutation.

5. Procédé pour la mesure de fronts d'onde produits par une lentille pour un oeil comportant les étapes suivantes :
- émission d'un rayonnement de mesure sur la lentille et
- détection de fronts d'onde du rayonnement de mesure après interaction avec la lentille,
- la détection d'un rayonnement de mesure après interactions avec la lentille s'effectuant à une fréquence de balayage pour le moins égale à la fréquence à laquelle se produisent les modifications de fronts d'onde dans le rayonnement de mesure après interaction avec la lentille, et
- le rayonnement de mesure étant cadencé à une fréquence de 100 Hertz ou plus, et
- un rayonnement de mesure produit à des longueurs d'onde se modifiant selon la fréquence d'émission du rayonnement étant dirigé sur la lentille, et
- le balayage effectué lors de la détection de fronts d'onde du rayonnement de mesure s'effectuant à une fréquence de balayage pour le moins égale à la fréquence d'émission du rayonnement.

6. Procédé selon la revendication 5, dans le cadre duquel
- il est généré un stimulus pour provoquer des modifications dynamiques de la lentille, et
- la détection des fronts d'onde s'effectue à une fréquence de balayage qui est pour le moins égale à la fréquence des modifications à provoquer.

7. Procédé selon l'une des revendications 5 à 6, dans le cadre duquel
- la détection de fronts d'onde s'effectue à une fréquence de balayage d'au moins 70 Hertz, et/ou
- le rayonnement de mesure est amplifié après interaction avec la lentille, et/ou
- le rayonnement de mesure est amplifié après interaction avec la lentille, l'amplification s'effectuant en amont de la détection au moyen de la fréquence de balayage et/ou
- pour détecter des fronts d'onde, un rayonnement de mesure est, après interaction avec la lentille, représenté au moyen d'un agencement de lentilles, et/ou
- il est généré un rayonnement de mesure présentant une puissance de rayonnement maximale prédéfinie pour la lentille et/ou pour l'oeil, et/ou
- il est, pour générer le rayonnement de mesure, utilisé au moins un rayonnement laser ou une diode superluminescente d'une longueur d'onde prédéfinie, et/ou
- il est généré un rayonnement de mesure d'au moins une longueur d'onde comprise entre 400 et 1000 nm.

8. Procédé selon l'une des revendications 5 à 7, dans le cadre duquel
- il est, pour générer le rayonnement de mesure, utilisé au moins deux rayonnements laser ou au moins deux diodes superluminescentes qui sont dirigés sur la lentille selon une fréquence de commutation, et
- il est, pour détecter des fronts d'onde, utilisé une fréquence de balayage qui est pour le moins égale à la fréquence de commutation.

9. Utilisation du dispositif selon l'une des revendications 1 à 4 pour la mesure des modifications d'accommodation dynamiques de la lentille d'un oeil.

10. Utilisation du dispositif selon l'une des revendications 1 à 4 pour la mesure d'aberrations chromatiques d'un oeil.

11. Utilisation du dispositif selon l'une des revendications 1 à 4 pour la mesure de la dispersion d'un verre de contact ou d'une lentille intraoculaire pour un oeil.

12. Utilisation du dispositif selon l'une des revendications 1 à 4 pour la mesure des influences optiques du mouvement d'un verre de contact ou d'une lentille intraoculaire par rapport au reste de l'oeil qui en est pourvu.

13. Utilisation du dispositif selon l'une des revendications 1 à 4 pour la mesure des influences optiques d'une modification du film lacrymal.

14. Utilisation du dispositif selon l'une des revendications 1 à 4 pour la détection et/ou la correction des aberrations d'une lentille intraoculaire d'un oeil.
